Europäisches Patentamt

European Patent Office

Office européen des brevets

(19)

(11) Veröffentlichungsnummer: **0 038 903**
**A2**

(12) # EUROPÄISCHE PATENTANMELDUNG

(21) Anmeldenummer: **81100523.0**

(22) Anmeldetag: **24.01.81**

(51) Int. Cl.³: **A 61 F 1/03**

(30) Priorität: **28.04.80 CH 3255/80**

(43) Veröffentlichungstag der Anmeldung:
**04.11.81 Patentblatt 81/44**

(84) Benannte Vertragsstaaten:
**AT DE FR GB IT NL**

(71) Anmelder: **GEBRÜDER SULZER**
**AKTIENGESELLSCHAFT**
**Zürcherstrasse 9**
**CH-8401 Winterthur(CH)**

(72) Erfinder: **Frey, Otto**
**Walrütistrasse 56**
**CH-8400 Winterthur(CH)**

(74) Vertreter: **Dipl.-Ing. H. Marsch Dipl.-Ing. K. Sparing**
**Dipl.-Phys.Dr. W.H. Röhl**
**Rethelstrasse 123**
**D-4000 Düsseldorf(DE)**

(54) Im Becken verankerbare Gelenkpfanne.

(57) Der kegelstumpfförmige Aussenmantel (1) der Gelenkpfanne ist mit entlang seiner Mantellinie (3) verlaufenden
reliefartigen Wulsten (4) versehen, die eine rotationsgesicherte, gegen elastische Verformung des Beckenknochens
nachgiebige Verankerung ermöglichen.

# Fig.2

EP 0 038 903 A2

Croydon Printing Company Ltd.

Gebrüder Sulzer, Aktiengesellschaft, Winterthur/Schweiz

Im Becken verankerbare Gelenkpfanne

Die Erfindung betrifft eine im Becken verankerbare Gelenkpfanne zur Aufnahme eines kugeligen Gelenkkopfes einer Hüftgelenkprothese, wobei der im Becken zu verankernde Aussenmantel mindestens teilweise eine Rotationsfläche, z.B. eine Kegelstumpffläche, ist.

Gelenkpfannen, deren Aussenmantel eine konisch verlaufende Kegelstumpffläche bildet, sind bekannt (siehe z.B. "Knochenersatz aus Keramik", Bild 3, auf Seite 276 der Zeitschrift "Umschau in Wissenschaft und Technik" 75 (1975), Heft 9, Seite 274-278). Auf ihrem Kegelstumpfmantel ist bei diesen bekannten Gelenkpfannen, die auch aus Kunststoff, beispielsweise aus Polyäthylen, gefertigt werden, ein Gewinde eingeschnitten, mit dem sie in den Beckenknochen eingeschraubt werden.

Während des Gehens treten im Becken bekanntlich elastische Verformungen auf, bei denen sich der Hohlraum des Verankerungsbettes für die künstliche Gelenkpfanne erweitert und wieder zusammenzieht. Diese Bewegungen, denen die bekannte Pfannenkonstruktion nicht zu folgen vermag, können zu Lockerungen dieser Pfannen führen, zumal die Verankerungsoberfläche relativ klein ist, da in den eingeschnittenen Gewindegängen immer nur die halbe Fläche trägt.

Aufgabe der Erfindung ist es, eine Gelenkpfanne der genannten Art zu schaffen, bei der die Gefahr der Lockerungen zumindest vermindert, wenn nicht ganz vermieden ist. Diese Aufgabe wird dadurch gelöst, dass aus dem rotationssymmetrischen Aussenmantel, über den Umfang verteilt und entlang einer Mantellinie verlaufend, Verankerungswulste reliefartig hervorspringen.

Die neue Gelenkpfanne wird im Becken in ein an ihren Aussenmantel - gegebenenfalls unter Berücksichtigung von zusätzlich einzubringendem Knochenzement - angepasstes Bett eingesetzt, wobei die Verankerung zementfrei, zementarm oder in einem vollständigen Knochenzementbett erfolgen kann. Die glatten Wände ihres Aussenmantels und der Verlauf der die Rotationssicherung übernehmenden Wulste entlang von Mantellinien des Kegelstumpfes ermöglichen der neuen Gelenkpfanne elastischen Erweiterungen und Verengungen ihres Bettes zu folgen und sich dabei in gewissem Umfang in den Knochen hinein und aus ihm heraus zu bewegen. Darüberhinaus besitzt die neue Gelenkpfanne am Aussenmantel mit den Wulsten eine relativ grosse Verankerungsfläche, so dass die auf sie wirkenden Kräfte weitgehend verteilt werden.

Diese Verankerungsfläche kann noch vergrössert werden, wenn die Verankerungswulste quer zur Mantellinie verlaufende Rippen in ihrer Oberfläche haben, zwischen die gegebenenfalls auch spongioses Gewebe einwachsen kann.

Die Verteilung der Wulste auf dem Umfang der Mantelfläche des Kegelstumpfes kann rotationssymmetrisch, aber auch unsymmetrisch erfolgen; dabei ergibt sich im letzteren Falle - beispielsweise für Gelenkpfannen, bei denen der Rand der den Gelenkkopf aufnehmenden, hohlkugelförmigen Pfannenschale unsymmetrisch verläuft, indem er einseitig erhöht ist - von selbst eine eindeutige Einbaulage, die bei rotationssymmetrischer Anordnung der Wulste erst durch eine exzentrische Markierung, beispielsweise einen exzentrischen Zapfen, hergestellt werden kann. Die neue Gelenkpfanne kann aus allen in der Implantattechnik verwendeten Materialien, wie Metallen, Kunststoffen oder Keramik und pyrolytischem Kohlenstoff, gefertigt werden.

Im folgenden wird die Erfindung anhand eines Ausführungsbeispiels im Zusammenhang mit der Zeichnung näher erläutert.

Fig. 1 zeigt eine Seitenansicht eines Ausführungsbeispiels der neuen Gelenkpfanne, das mit einem unsymmetrischen Rand für die eigentliche hohlkugelförmige Pfannenschale versehen ist;

Fig. 2 ist eine Aufsicht auf die Gelenkpfanne in Richtung der Rotationsachse ihres Aussenmantels.

Der Aussenmantel 1 der Gelenkpfanne ist im Grundkonzept eine konische Kegelstumpffläche, deren Symmetrieachse mit 2 bezeichnet ist. Aus seiner Mantelfläche springen reliefartig entlang von Mantellinien 3 des Aussenmantels 1 verlaufende Verankerungswulste 4 hervor, die gleichmässig über den Umfang verteilt sind und bezüglich der Symmetrieachse 2 eine 6-zählige Symmetrie aufweisen. Quer zur Mantellinie 3 sind die Oberflächen der einzelnen Verankerungswulste 4 mit Rippen 5 versehen; die Zwischenräume zwischen den Rippen 5 dienen zur zusätzlichen Vergrösserung der Verankerungsoberflächen und/oder dem Einwachsen von spongiosem Knochengewebe.

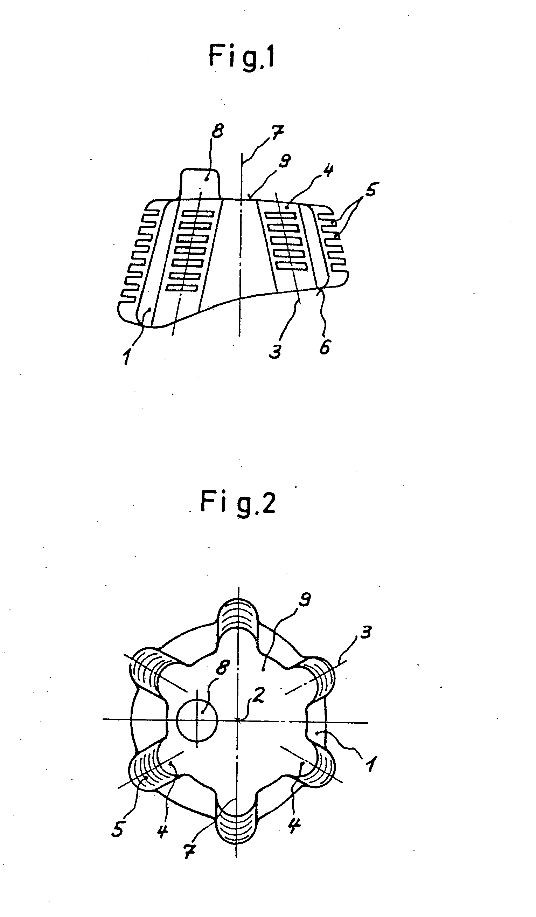

Der die eigentliche hohlkugelförmige Pfannenschale begrenzende Rand 6 der Gelenkpfanne ist, wie Fig. 1 erkennen lässt, unsymmetrisch ausgebildet, d.h. bezüglich einer Mittelebene 7 durch die Symmetrieachse 2 auf einer Seite weiter heruntergezogen als auf der anderen. Dadurch wird es notwendig, dass die Gelenkpfanne eine bestimmte Einbaulage im Beckenknochen einnimmt. Da ihr Aussenmantel 1 jedoch rotationssymmetrisch aufgebaut ist, ist als Markierung für die Festlegung dieser Einbaulage ein exzentrisch angeordneter Zapfen 8 auf der oberen Basisfläche 9 des kegelstumpfförmigen Aussenmantels 1 vorgesehen.

Wie bereits erwähnt, erfolgt die Verankerung der neuen Gelenkpfanne im Beckenknochen entweder zementfrei, zementarm - d.h. nur Teilflächen des Aussenmantels 1 sind in Knochenzement eingebettet - oder auf konventionelle Weise in einem geschlossenen Zementbett. Vor dem Einsetzen der Gelenkpfanne wird der Knochen mit einem, an die Pfannenform mit ihren reliefartigen Wulsten 4 und gegebenenfalls mit ihrem Markierungszapfen 8 angepassten Instrument operativ entsprechend vorbereitet, wobei bei dem im Knochen geschaffenen Pfannenlager selbstverständlich berücksichtigt wird, welche der vorstehend erwähnten Verankerungsarten gewählt wird. Die erfindungsgemässe Aussenform der Pfanne ermöglicht dann eine rotationsgesicherte grossflächige Abstützung der Gelenkpfanne im Beckenknochen, die den elastischen Verformungen des Beckens bei Be- und Entlastungen in gewissem Umfang nachgeben kann.

Patentansprüche

1. Im Becken verankerbare Gelenkpfanne zur Aufnahme eines kugeligen Gelenkkopfes einer Hüftgelenkprothese, wobei der im Becken zu verankernde Aussenmantel mindestens teilweise eine Rotationsfläche, z.B. eine Kegelstumpffläche, ist, dadurch gekennzeichnet, dass aus dem rotationssymmetrischen Aussenmantel (1), über den Umfang verteilt und entlang einer Mantellinie (3) verlaufend, Verankerungswulste (4) reliefartig hervorspringen.

2. Gelenkpfanne nach Anspruch 1, dadurch gekennzeichnet, dass der Rand (6) der den Gelenkkopf aufne enden, hohlkugelförmigen Pfannenschale unsymmetrisch verläuft, indem er einseitig erhöht ist.

3. Gelenkpfanne nach Anspruch 1, dadurch gekennzeichnet, dass die Verankerungswulste (4) rotationssymmetrisch auf dem Umfang des Aussenmantels verteilt sind.

4. Gelenkpfanne nach Anspruch 2 und 3, dadurch gekennzeichnet, dass der Aussenmantel (1) eine exzentrische Markierung (8) zur Festlegung einer eindeutigen Einbaulage für die Gelenkpfanne hat.

5. Gelenkpfanne nach Anspruch 4, dadurch gekennzeichnet, dass die Markierung aus einem exzentrischen Zapfen (8) besteht.

6. Gelenkpfanne nach Anspruch 1, dadurch gekennzeichnet, dass die Verankerungswulste (4) quer zur Mantellinie (3) verlaufende Rippen (5) in ihrer Oberfläche haben.

Fig.1

Fig.2